# EUROPEAN PATENT APPLICATION

(11) **EP 4 131 277 A1**
(43) Date of publication of application: **08.02.2023**
(21) Application number: 21774332.7
(22) Date of filing: 26.03.2021
(51) Int. Cl.: G16H 10/40, G16H 50/20

(54) **INFORMATION PROCESSING DEVICE, EXAMINATION SYSTEM, CONTROL METHOD FOR INFORMATION PROCESSING DEVICE, CONTROL PROGRAM, AND RECORDING MEDIUM**

(30) Priority: 27.03.2020 JP 2020058363
(71) Applicant: Kyocera Corporation, Kyoto-shi Kyoto 612-8501 (JP)
(72) Inventor: SATO, Masaki, Kyoto-shi, Kyoto 612-8501 (JP)
(74) Representative: TBK
(86) International application number: PCT/JP2021/012807
(87) International publication number: WO 2021/193904

(57) **Abstract**

To provide a user with a test result of a viral infection or a bacterial infection based on detection data obtained by detecting particles of the virus or bacteria from the user even if the user has not been tested in a hospital or the like. An information processing device includes a test request reception unit receiving, from a communication terminal, test request information including detection data obtained by a detection device that detects particles of virus or bacteria contained in a specimen collected from a user, an infection calculation unit calculating an infection state of the user based on the detection data, and a test result output unit generating test result information including a calculation result from the infection calculation unit and outputting the test result information to the communication terminal.

## Description

### TECHNICAL FIELD

The present disclosure relates to an information processing device, a test system, and a control method for the information processing device for presenting test results regarding viral infections or bacterial infections.

### BACKGROUND OF INVENTION

There are many cases in which, if a viral infection or a bacterial infection is found at an early stage and a treatment starts from the initial stage of the infection, the onset of the disease can be avoided or development of the disease to a severe condition can be curbed. To this end, a person needs to visit a medical institution, a test institution, etc. to get a predetermined test at the stage with no onset or when symptoms are mild. However, because the number of particles of virus or bacteria in a specimen collected from an infected person experiencing the stage with no onset or showing mild symptoms is small, there is a possibility of the test result of the person being negative in a single test even though the person is actually infected.

Patent Document 1 discloses a technology that is available to detect particles of virus such as influenza virus with high sensitivity.

### CITATION LIST

### PATENT LITERATURE

Patent Document 1: JP 2018-038384 A

### SUMMARY

In an embodiment of the present disclosure, an information processing device includes a test request reception unit configured to receive, from a communication terminal, test request information including detection data obtained by a detection device that detects a detection target object included in a specimen collected from a user, an infection calculation unit configured to calculate an infection state of the user based on the detection data, and a test result output unit configured to generate test result information including a calculation result from the infection calculation unit and transmit the test result information to the communication terminal.

In an embodiment of the present disclosure, a control method for an information processing device includes receiving, from a communication terminal, test request information including detection data obtained by a detection device configured to detect a detection target object contained in a specimen collected from a user;
calculating an infection state of the user based on the detection data; and
generating test result information including a calculation result in the infection calculation and transmitting the test result information to the communication terminal.

An information processing device according to each aspect of the present disclosure may be implemented by a computer, and in this case, a control program for an information processing device that causes the computer to realize the information processing device by causing the computer to operate as each unit (software element) provided in the information processing device, and a computer readable recording medium in which the control program is recorded also fall within the scope of the present disclosure.

### BRIEF DESCRIPTION OF THE DRAWINGS

FIG. 1 is a diagram illustrating a configuration example of a test system according to a first embodiment of the present disclosure.
FIG. 2 is a block diagram illustrating an example of a configuration of a detection device and a communication terminal.
FIG. 3 is a diagram illustrating a configuration example of a specimen attachment part.
FIG. 4 is a diagram illustrating an example of test request information.
FIG. 5 is a diagram illustrating an example of movement history information.
FIG. 6 is a block diagram illustrating an example of a configuration of a communication terminal.
FIG. 7 is a block diagram illustrating an example of a configuration of an information processing device and a storage device according to the first embodiment of the present disclosure.
FIG. 8 is a diagram illustrating an example of a data structure of a user database.
FIG. 9 is a diagram illustrating an example of a data structure of a test request information database.
FIG. 10 is a diagram illustrating an example of test result information.
FIG. 11 is a diagram illustrating an example of a data structure of a result database.
FIG. 12 is a diagram illustrating an example of a data structure of a movement history database.
FIG. 13 is a diagram illustrating an example of a risk map.
FIG. 14 is a sequence diagram showing an example of the flow of a process in which the information processing device that has received the test request information from a communication terminal transmits test result information and then creates a risk map.
FIG. 15 is a flowchart showing an example of a flow of an infection state calculation process executed by the information processing device.
FIG. 16 is a flowchart showing an example of the flow of a result information generation process executed by the information processing device.
FIG. 17 is a sequence diagram showing an example of the flow of a process performed by the information processing device to distribute infection information in response to an information distribution request from a communication terminal.
FIG. 18 is a diagram illustrating a configuration example of a test system according to a second embodiment of the present disclosure.

### DESCRIPTION OF EMBODIMENTS

### First Embodiment

### Overview of Information Processing Device 1

An information processing device 1 according to one aspect of the present disclosure calculates the infection state of a corresponding user upon receiving test request information from a communication terminal 2. The test request information includes detection data obtained by a detection device 3 that detects particles of a virus (a detection target object) or bacteria (a detection target object) contained in a specimen 5 collected from the user. The information processing device 1 generates test result information including the infection state of the user calculated based on the detection data, and transmits the test result information to the communication terminal 2. The detection device 3 may detect a fungus (a detection target object), Rickettsia (a detection target object), or Spirochaetes (a detection target object).

Here, an infection state may be a determination result of whether a user has already been infected with a virus or bacteria, or information indicating a possibility of a user being infected with a virus or bacteria.

According to the configuration described above, the information processing device 1 determines an infection state indicating whether a user has been infected with a virus or bacteria based on test request information received from the communication terminal 2, and transmits the determination result to the communication terminal 2. The user can, for example, provide a specimen 5 collected from himself or herself at home, for example, to the detection device 3. In this way, the user can receive the determination result of whether the user has been infected with a virus or bacteria without visiting a test institution such as a hospital. Note that this does not exclude carrying out a test at a test institution such as a hospital, and tests may be carried out at a test institution such as a hospital.

The information processing device 1 can generate test result information regarding infection with a variety of viruses or bacteria as long as the viruses and bacteria can be detected by the detection device 3, and can provide the user with the test result information. The detection device 3 is not limited thereto, but is capable of detecting the viruses or bacteria described below, for example.

(Examples of detectable viruses): influenza virus, corona virus, severe acute respiratory syndrome (SARS) corona virus, Middle East respiratory syndrome (MERS) virus, mumps virus, measles virus, Nipah virus, canine distemper virus, human immunodeficiency virus (HIV), hepatitis B virus, human T-cell leukemia virus (HTLV), Ebola virus, hepatitis C virus, Lassa virus, Hanta virus, rabies virus, Japanese encephalitis virus, yellow fever virus, dengue virus, rubella virus, rotavirus, herpes virus, norovirus, and the like.

(Examples of detectable bacteria): Yersinia pestis, Shigella, Vibrio cholerae, Salmonella typhi, Diphtheria, enterohemorrhagic Escherichia coli (e.g., E.coli 0157, etc.), fulminant hemolytic streptococcus, anthrax, Clostridium tetani, Brucellosis, Legionella pnuemophila, meningococcus, drug-resistant Pseudomonas aeruginosa, methicillin-resistant Staphylococcus aureus (MRSA), tularemia, Spirillum minus, gram-negative facultative anaerobic bacilli of Streptobacillus, ringworm, Candida fungus (e.g., Candida albicans), Aspergillus fungus, Cryptococcus neoformans, Bartonella fungus (e.g., Bartonella bacilliformis), Mycobacterium tuberculosis, gonorrhea, Vancomycin-resistant cocci, Penicillin-resistant Streptococcus pneumoniae, Streptococcus pneumoniae, Haemophilus influenzae, and the like.

Hereinafter, a case in which the detection device 3 is a device for detecting particles of the influenza virus will be described as an example. In this case, the information processing device 1 generates test result information including the determination result of whether a user has been infected with the influenza virus and transmits the test result information to the communication terminal 2. In the following, the influenza virus may simply be referred to as "the virus".

The specimen 5 may, when a user is suffering from the infectious disease, be an arbitrary one containing particles of the virus and a component derived from the virus causing an infectious disease. The specimen 5 may be one that can be collected from a user in a non-invasive treatment, for example, saliva, sputum, a nasal drip, or the like of the user, or one that can be collected from a user in a minimal invasive treatment, such as blood, a tissue sample, or the like.

If particles of the virus can be detected with high sensitivity, for example, the specimen 5 may be some of a liquid spat out by a user after the user has gargled with a predetermined amount (e.g., 2 ml) of the liquid for a predetermined amount of time (e.g., 5 seconds). In this case, after gargling, the user may spit out the liquid used after the gargle to a container and provide some of the liquid to a specimen attachment part 321 to be described below. Particles of the virus contained in the saliva and sputum of the user can be easily collected by the user from the liquid used for the gargle.

The specimen attachment part 321 may be given different identification numbers depending on the virus species to be detected. Alternatively, when the specimen attachment part 321 is mounted in the detection device 3, the detection device 3 may be configured to recognize which virus detection the specimen attachment part 321 is used. The specimen attachment part 321 may detect one type of virus or type of bacteria, or may detect a plurality of types of viruses or a plurality of types of bacteria.

### Configuration of Test System 100

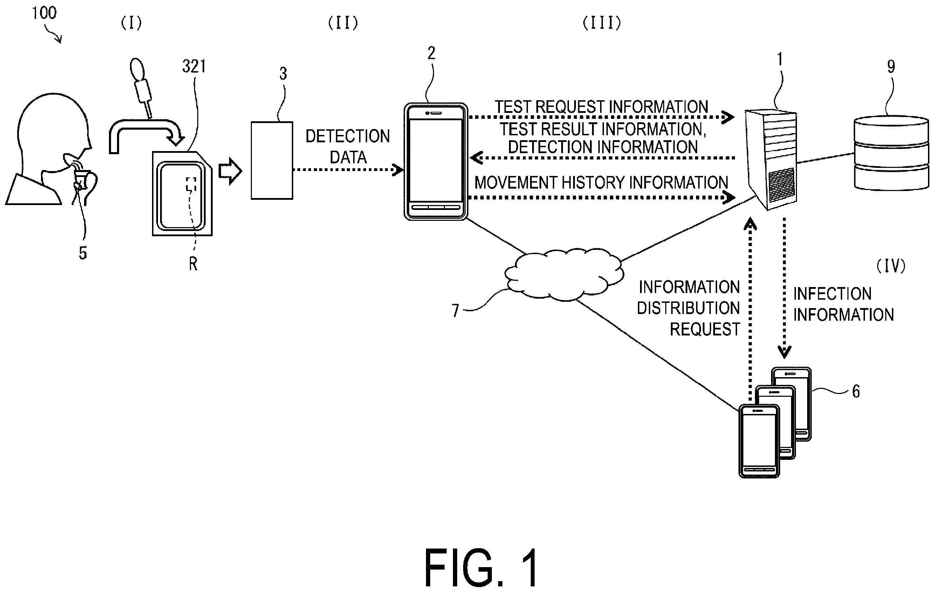

First, a configuration of a test system 100 according to an aspect of the present disclosure will be described using FIG 1. FIG. 1 is a diagram illustrating a configuration example of the test system 100 in which the information processing device 1 is introduced.

The test system 100 includes the detection device 3 that detects particles of the influenza virus contained in the specimen 5 collected from a user, the information processing device 1, and the communication terminal 2 communicatively coupled to the detection device 3 and the information processing device 1. The test system 100 may further include a communication terminal 6 that receives infection information distributed from the information processing device 1. The information processing device 1, the communication terminal 2, and the communication terminal 6 are communicatively coupled via a communication network 7. The infection information may be, for example, information including the number of infected people in each area. Alternatively, the infection information may be a risk map representing a time and an area in which a virus-infected person is likely to release the virus having infectivity on a map.

A user who desires to use the test system 100 submits registration information to the information processing device 1 in advance as a user of the test system 100. For example, the submission of the registration information may be received by distributing a web page for allowing the user to input the registration information from the information processing device 1 to the communication terminal 2 to allow the user to input required matters. The registration information may be transmitted from the communication terminal 2 to the information processing device 1 by e-mail or by using an application installed in the communication terminal 2. Alternatively, the submission of the registration information may be accepted by having each user who wants to use the test system 100 mail the registration information in a paper medium to the administrator of the information processing device 1, for example. The user registered as a user of the test system 100 may be given a user ID that is identification information unique to each user.

Information to be input by the user as the registration information may include personal information of the user. The personal information input as the registration information may include contact information such as the address, name, attributes, telephone number, and mail address of the user. The attributes may include the age and gender of the user. For example, if the user is a minor, the personal information input as the registration information may include the address, name, age, occupation, and mail address of the user's family (e.g., the parents).

The personal information input by the user as the registration information may include information regarding the method of payment by the user for paying a fee to use the test system 100. The information regarding the method of payment may include credit card information of the user.

The information processing device 1 stores, for each user, registration information in the storage device 9 in association with the user ID given to the user. The information processing device 1 may store the date when registration information was received, in the storage device 9 for each user. In this specification, a user registered as a user of the test system 100 is simply referred to as the "user".

The user may be able to receive a desired service among the service A and service B described below. As an example, the test system 100 may allow each user to select any service to use among the following service A and service B described below at any time.
- Service A: To receive the test result regarding viral infection from the information processing device 1.
- Service B: To receive infection information distributed from the information processing device 1.

The user possesses the detection device 3. For example, the administrator of the information processing device 1 who has received a predetermined price or payment for a predetermined rental fee may mail the detection device 3 to the user. Alternatively, the user may purchase the detection device 3 from a store or the like. If the detection device 3 has been rented to the user, the administrator of the information processing device 1 may request taking the procedure to change the period in which the detection device 3 is supposed to be lent and the price for the changed period to the user within the aforementioned period. The procedure to change the period requested from the user may be, for example, an extension procedure.

The identification information (e.g., device ID) assigned to the detection device 3 possessed by the user may be registered (input) by the user. In such a case, the information processing device 1 may store the device ID acquired from each user in the storage device 9 for each user.

The test system 100 includes the steps (I) to (IV) described below, as illustrated in FIG. 1.

### (I) Specimen collection step

First, the user collects a specimen 5 from themselves. The collected specimen 5 is provided to the specimen attachment part 321 included in the detection device 3. The specimen attachment part 321 provided with the specimen 5 is mounted in the detection device 3.

### (II) Detection Step

The detection device 3 detects particles of the virus contained in the specimen 5 collected from the user, and transmits the detection data to the communication terminal 2.

### (III) Test Result Providing Step

The communication terminal 2 that has received the detection data from the detection device 3 generates test request information using the detection data, and transmits the test request information to the information processing device.

Upon receiving the test request information, the information processing device 1 generates test result information based on the detection data and the like included in the test request information. The information processing device 1 transmits the test result information to the communication terminal 2 that is the transmission source of the test request information.

The information processing device 1 may generate detection information from an image taken of the specimen attachment part 321 when the test request information includes such an image. The detection information may be information including the number of particles of the virus in a predetermined area on the specimen attachment part 321. The information processing device 1 transmits the detection information to the communication terminal 2 that is the transmission source of the test request information.

The user that has been presented with the generated detection information confirms that particles of the virus have been detected from the specimen collected from themselves and realizes that they are infected with the virus. In this way, the generation of the detection information and presentation thereof to the user can promote the user infected with the virus to take early measures and treatments.

If the test request information includes information essential to the diagnosis based on predetermined diagnostic criteria, the test result information may include a diagnostic result based on the predetermined diagnostic criteria. In this case, the test result information may only present the diagnostic criteria. Alternatively, the test result information may be used to help a doctor to confirm the test result and to present the diagnostic result from the doctor. With this configuration, the user can receive the diagnostic result while being at home without having to visit a medical facility or the like to get diagnosed.

The information processing device 1 may include a neural network that outputs a diagnostic result for a viral infection in response to input of information such as detection data included in test request information. The neural network may be subjected to machine learning in advance. Learning data used for the machine learning may include, for example, test results obtained by providing specimens collected from a plurality of patients who were diagnosed by doctors in medical facilities to the detection device 3. The test results obtained by providing specimens collected from a plurality of patients who were diagnosed by doctors in medical facilities to the detection device 3 may be, for example, data regarding virus detection. Training data used for the machine learning may be diagnosis results of a doctor's diagnosis of each patient as to whether they are suffering from a viral infection.

The information processing device 1 may acquire movement history information of the user from the communication terminal 2. For example, when the test result regarding viral infection is positive, the information processing device 1 may transmit a movement history request together with the test result information including the test result. For the movement history information, for example, the position information of each time stored in the communication terminal 2 may be used. The movement history information may be information created by the communication terminal 2 in which a movement history of the user is displayed on a map. The movement history information may be, for example, information indicating a movement history of the user in a predetermined period of time before and after the time point at which the user collected his or her specimen to transmit test request information. The predetermined period of time may be a period of time including the time when the user was infected with the virus and the time until the viral infection is completely cured. For example, the predetermined period of time may be the period of time including 7 days before the specimen is collected and 7 days after the specimen was collected.

The information processing device 1 may store the received test request information, and the test result information corresponding to each piece of the test request information in the storage device 9. The information processing device 1 may also store the acquired movement history information in the storage device 9.

Here, for communication between the information processing device 1 and the communication terminal 2, encrypted communication such as a Secure Socket Layer (SSL) may be used as a security measure. This makes it possible to safely transmit and receive the registration test request information, and the test result information including the personal information of the user.

### (IV) Infection Information Distribution Step

The information processing device 1 may generate the infection information based on the test result information and the like generated in accordance with the test request information from a plurality of users.

Upon receiving an information distribution request from the communication terminal 6, the information processing device 1 distributes the infection information to the communication terminal 6. The communication terminal 6 is a communication terminal possessed by a user using the test system 100. The communication terminal 6 may have substantially the same functions as the communication terminal 2. In the present specification, a terminal transmitting test request information will be referred to as "the communication terminal 2", and a terminal transmitting information distribution request will be referred to as "the communication terminal 6".

When the user of the communication terminal 2 that transmits the test request information needs to be distinguished from the user of the communication terminal 6 that transmits the information distribution request, the former may simply be referred to as the "user", and the latter may be referred to as the "second user" below. However, the user and the second user are both registered as users of the test system 100, and there is no substantial difference between them.

### Detection Device 3 and Communication Terminal 2

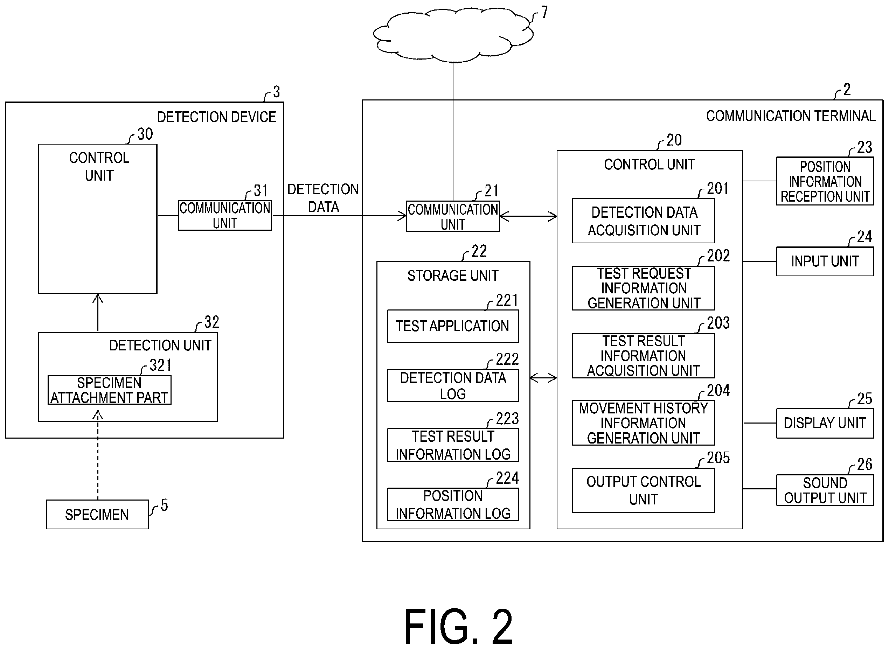

A configuration of the detection device 3 and the communication terminal 2 will be described using FIG. 2. FIG. 2 is a block diagram illustrating an example of a configuration of the detection device 3 and the communication terminal 2.

### Configuration of Detection Device 3

First, a configuration of the detection device 3 will be described. The detection device 3 detects particles of the virus contained in the specimen 5 collected from the user. The detection device 3 may be installed in the house of the user, installed in a facility such as a school or factory, or installed in a test institution such as a hospital, or may be carried by a user.

The detection device 3 includes a control unit 30 that controls various processing operations executed by the detection device 3, a communication unit 31 that communicates with the communication terminal 2, and a detection unit 32 with the specimen attachment part 321. The detection unit 32 detects particles of the virus contained in the specimen 5 provided in the specimen attachment part 321. The detection unit 32 outputs the detection result to the control unit 30. Here, the detection result may include information identifying the position, number, and proportion of a specimen embedding part 322 in which a reaction product has been produced.

The control unit 30 acquires the detection result from the detection unit 32, and generates the detection data based on the detection result. The detection data generated by the control unit 30 is transmitted to the communication terminal 2 via the communication unit 31. The detection data may include an image taken of at least a portion of the specimen attachment part 321.

Communication between the communication terminal 2 and the detection device 3 may be short-range wireless communication or communication by wired connection. Alternatively, the detection device 3 and the communication terminal 2 may be directly coupled with a connector such as a USB terminal.

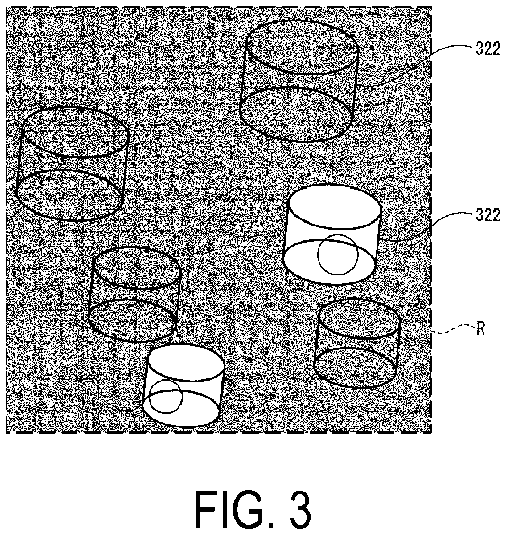

FIG. 3 is a diagram illustrating a configuration example of the specimen attachment part 321. FIG. 3 is an enlarged schematic diagram illustrating a region R of the specimen attachment part 321 illustrated in FIG. 1. FIG. 3 illustrates the region R of the specimen attachment part 321 as viewed from the back side to which the specimen is attached. In one example, the specimen attachment part 321 has a plurality of specimen embedding parts 322 for fixing and detecting particles of the virus contained in the specimen 5. The specimen embedding part 322 is a recessed portion formed on the main surface of the specimen attachment part 321. The specimen embedding part 322 may be, for example, a cylindrical recessed portion. The volume manageable by the specimen embedding part 322 is, for example, from 10 aL to 100 nL. The specimen attachment part 321 may be a disposable and detachable cartridge of the detection device 3. The specimen attachment part 321 is housed in the detection unit 32.

A detection target object may be detected, for example, by detecting luminescence in each specimen embedding part 322. The luminescence in each specimen embedding part 322 may be, for example, fluorescent or phosphorescent. The luminescence in each specimen embedding part 322 may be light emission, for example, based on a reaction product produced from an enzymatic reaction. The luminescence in each specimen embedding part 322 may be luminescence, for example, based on a luminescent substance that interacts with nucleic acid amplified using a nucleic acid amplification method.

Each specimen embedding part 322 includes a substrate (e.g., 4-methylumbelliferyl-α-D-neuraminic acid) that reacts with an enzyme (e.g., neuraminidase) present on the particle surfaces or inside the particles of the virus. The substrate may be immobilized on an inner wall of the specimen embedding part 322 using any known technique. When specimen 5 is provided to the specimen attachment part 321, the specimen embedding part 322 in which particles of the virus are present and the specimen embedding part 322 in which no particles of the virus are present are generated. Within the specimen embedding part 322 in which particles of the virus are present, a reaction product (e.g., 4-methylumbelliferone) is produced from the reaction of the enzyme present on the particle surfaces or inside the particles of the virus with the substrate contained in the specimen embedding part. On the other hand, no reaction product is produced in the specimen embedding part 322 in which no particles of the virus are present. As the number of particles (concentration) of the virus contained in the specimen 5 increases, the number of specimen embedding parts 322 in which the reaction product is produced increases.

The reaction product may be, for example, a molecule that emits light with a characteristic wavelength in response to irradiation with light having a predetermined wavelength. In this case, the detection unit 32 may emit light having a predetermined wavelength toward the specimen attachment part 321, and detect fluorescence from the reaction product generated in the specimen embedding part 322.

To accurately calculate the infection state of the user infected with the virus, using the results obtained by detecting particles of the virus contained in each of specimens 5 collected at different times is desirable. Thus, the detection device 3 may output detection data including a first detection result obtained by detecting particles of the virus contained in a specimen 5 collected from the user at a first time, and a second detection result obtained by detecting particles of the virus contained in another specimen 5 collected from the user at a second time that is different from the first time. Here, the second time is intended to be any time after the first time. For example, the first detection result is a detection result output at 10 am on a certain day, and the second detection result may be a result output at 4 pm on the same day. Alternatively, the first detection result is a detection result output at 10 pm on a certain day, and the second detection result may be a result output at 9 am on the next day.

Alternatively, the detection device 3 may be configured to output detection data each time a specimen 5 is collected and the specimen 5 is provided to the specimen attachment part 321.

Information regarding a change over time in the number of particles of the virus in the specimen is useful for determining the infection state of the user. If the configuration described above is adopted, the information processing device 1 can accurately estimate an infection phase of the user. Infection phases will be described below.

### Configuration of Communication Terminal 2

A configuration of the communication terminal 2 will be described. The communication terminal 2 receives detection data from the detection device 3 and generates test request information including the detection data. The communication terminal 2 transmits the generated test request information to the information processing device 1.

The communication terminal 2 is a computer communicatively coupled to the detection device 3 and the information processing device 1, and for example, includes a smartphone and a tablet terminal that the user can carry while moving. Hereinafter, a case in which the communication terminal 2 is a smartphone will be described as an example.

The communication terminal 2 includes a control unit 20, a communication unit 21, and a storage unit 22 as illustrated in FIG. 2. The communication terminal 2 may further include a position information reception unit 23, the input unit 24, a display unit 25, and a sound output unit 26.

The control unit 20 is a CPU, for example, and performs control to execute processing of each function of the communication terminal 2. The control unit 20 includes a detection data acquisition unit 201, a test request information generation unit 202, a test result information acquisition unit 203, a movement history information generation unit 204, and an output control unit 205.

The storage unit 22 is a storage device in which various computer programs to be read by the control unit 20, data to be used in various processing operations executed by the control unit 20, and the like are stored. The storage unit 22 includes a test application 221, a detection data log 222, a test result information log 223, and a position information log 224.

The position information reception unit 23 periodically receives signals to be used for calculating the current position of the communication terminal. The position information reception unit 23 receives signals from, for example, a Global Navigation Satellite System (GNSS). The control unit 20 analyzes a received signal, generates position information indicating the current position of the communication terminal, associates the position information with information indicating the time and date on which the signal was acquired, and stores the associated information in the position information log 224 of the storage unit 22. The position information reception unit may receive signals from a system, for example, GPS, GLONASS, Galileo, BeiDou Navigation Satellite System, QZSS, the Junten Zenith Satellite, or the like.

The input unit 24 receives operations of inputting various types of information from the user. The input unit 24 may be, for example, a touch panel superimposed on a display screen of the display unit 25.

The detection data acquisition unit 201 acquires detection data from the detection device 3. The detection data acquisition unit 201 associates the detection data with information indicating the date and time on which the detection data was acquired, and stores the associated information in the detection data log 222 of the storage unit 22.

The test request information generation unit 202 generates test request information based on the detection data from the detection device 3. As a result, the information processing device 1 can generate highly reliable test result information based on the information included in the test request information, and provide the information to the user.

### Test Request Information

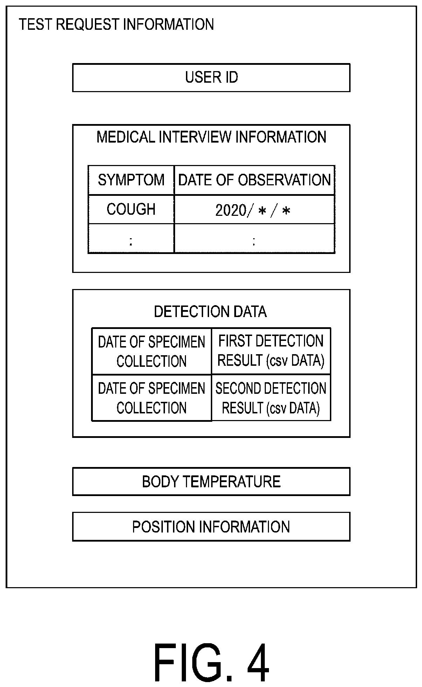

Here, the test request information will be described using FIG. 4. FIG. 4 is a diagram illustrating an example of the test request information.

The test request information may include the user ID, which is identification information unique to each user, each user's medical interview information, detection data, body temperature, and position information. In addition to the user ID or instead of the user ID, the test request information may include personal information such as the name, age, and gender of the user. The medical interview information may include information regarding the symptoms that have developed in the user and information indicating the date of observation on which the symptoms were observed.

The detection data includes the date and time on which the specimen was collected (a first time) and a detection result obtained by detecting the virus included in the specimen. In FIG. 4, a case of detection data including a first time and a first detection result, and a second time and a second detection result is exemplified. The detection results are detection data output by the detection device 3, and may be, for example, CSV data. The detection results may include an image taken of at least a portion of the specimen attachment part 321.

The body temperature and position information may respectively be the body temperature and position information of the user on the date and time on which the specimen was collected. The body temperature may be, for example, the body temperature measured at the first time and the second time, or may be the body temperature measured at the time when transmission of the test request information was prepared. The position information is information indicating the position of the communication terminal 2. The position information may be information indicating the position of the communication terminal 2 at the time when the test request information was transmitted. The position information may be latitude/longitude information calculated based on a signal received by the position information reception unit 23.

According to the configuration described above, the information processing device 1 can generate highly reliable test result information in accordance with the infection state of the user and provide the information to the user. Furthermore, if the test request information includes the position information of the user is included, the information processing device 1 can acquire information about a region where the viral infection is likely to spread, and a region where there are many virus-infected people. In this way, the information processing device 1 can generate and distribute infection information regarding viral infection.

Returning to FIG. 2, the test result information acquisition unit 203 acquires the test result information from the information processing device 1 via the communication network 7. The test result information acquisition unit 203 associates the acquired test result information with information indicating the date and time on which the test result information was acquired, and stores the associated information in the test result information log 223 of the storage unit 22. The test result information will be described below.

The movement history information generation unit 204 generates movement history information indicating a movement history of the user in a predetermined period of time based on the position information log 224 of the storage unit 22. The movement history information generation unit 204 may generate movement history information in response to a movement history request from the information processing device 1. The predetermined period of time may be a period of time a predetermined period of time before the transmission of the test request information from the communication terminal 2 to the information processing device 1 to the present.

### Movement History Information

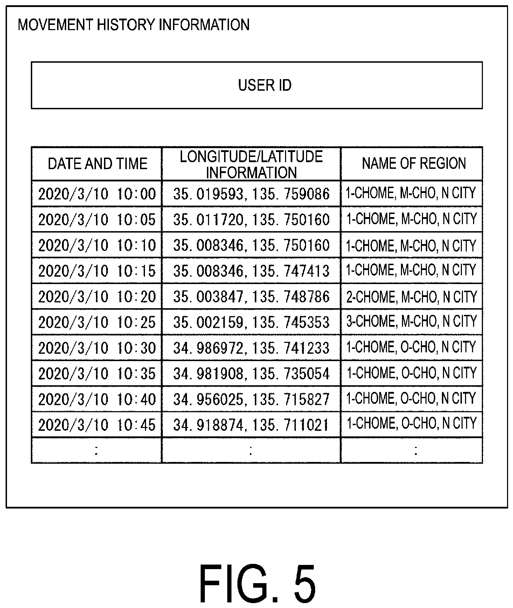

Here, the movement history information will be described using FIG. 5. FIG. 5 is a diagram illustrating an example of movement history information.

The movement history information includes a user ID as illustrated in FIG. 5. The movement history information may further include position information (e.g., latitude/longitude information) of the communication terminal 2 to the present, and the name of the region indicated by each piece of the latitude/longitude information.

The output control unit 205 controls the display unit 25 such that various types of information and images, and the like are displayed. Furthermore, the output control unit 205 controls the sound output unit 26 such that sound, an alarm, and the like are output.

### Configuration of Communication Terminal 6

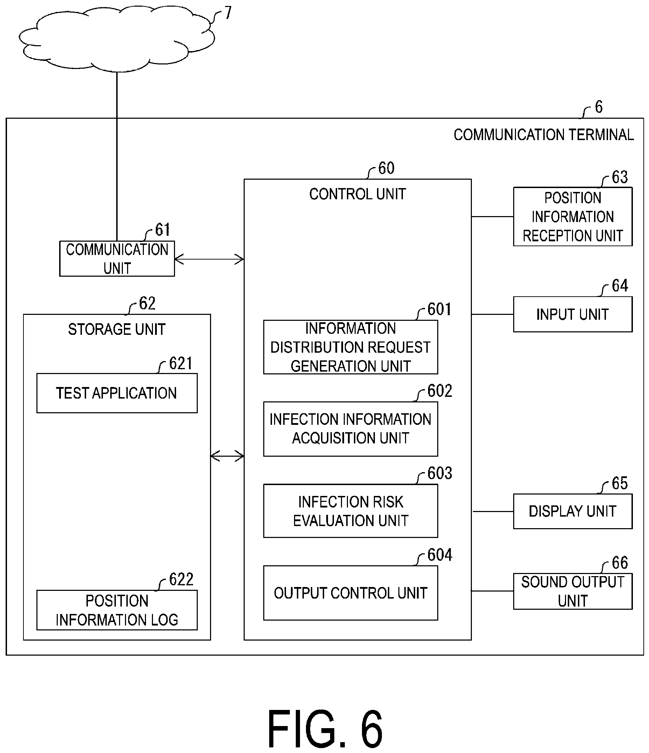

Subsequently, a configuration of the communication terminal 6 will be described using FIG 6. FIG. 6 is a block diagram illustrating an example of a configuration of the communication terminal 6. The communication terminal 6 transmits an information distribution request to the information processing device 1, and receives infection information from the information processing device 1. Although the communication terminal 2 and the communication terminal 6 are distinguished and described in the present specification, both terminals are communication terminals of users using the test system 100, and there is no substantial difference. In other words, both the communication terminal 2 and the communication terminal 6 may include all of the functions illustrated in FIGs. 2 and 6.

The communication terminal 6 is a computer communicatively coupled to the information processing device 1, and for example, includes a smartphone and a tablet terminal that a second user can carry while moving. Hereinafter, a case in which the communication terminal 6 is a smartphone will be described as an example.

The communication terminal 6 includes a control unit 60, a communication unit 61, and a storage unit 62. The communication terminal 6 may further include a position information reception unit 63, an input unit 64, a display unit 65, and a sound output unit 66.

The control unit 60 is a CPU, for example, and performs control to execute processing of each function of the communication terminal 6. The control unit 60 includes an information distribution request generation unit 601, an infection information acquisition unit 602, an infection risk evaluation unit 603, and an output control unit 604.

The storage unit 62 is a storage device in which various computer programs to be read by the control unit 60, data to be used in various processing operations executed by the control unit 60, and the like are stored. The storage unit 62 includes a test application 621 and a position information log 622.

The position information reception unit 63 has the same function as or a similar function to that of the position information reception unit 23 of the communication terminal 2.

The input unit 64 accepts input operations in which various types of information are input by the second user. The input unit 64 may be, for example, a touch panel superimposed on a display screen of the display unit 65.

The information distribution request generation unit 601 generates an information distribution request for requesting the information processing device 1 to distribute infection information. The information distribution request includes, for example, the user ID of the second user. Furthermore, the information distribution request may include, if necessary, at least one of information designating a region previously registered by the second user, information designating the region of which infection information is desired by the second user, and the position information of the communication terminal 6. The information distribution request is transmitted to the information processing device 1 via the communication unit 61.

The infection information acquisition unit 602 receives the infection information from the information processing device 1.

The infection risk evaluation unit 603 may calculate the infection risk for the second user based on the received infection information and the position information of the communication terminal 6. For example, the infection risk evaluation unit 603 may instruct the output control unit 604 to output an alarm, a message, or the like indicating the level of the infection risk, in accordance with the calculated infection risk. The infection risk evaluation unit 603 calculates that the infection risk is high if the second user has entered a region having a high risk of infection, and when the second user is already in a region having a high risk of infection as one example.

The infection risk evaluation unit 603 may calculate the infection risk for the second user based on the received infection information and the position information log 622 of the communication terminal 6. For example, the infection risk evaluation unit 603 may instruct the output control unit 604 to output an alarm, a message, or the like indicating the level of the infection risk, in accordance with the calculated infection risk. The infection risk evaluation unit 603 calculates that the infection risk is high if the second user passed through a region where the infection risk is high at a time when the infection risk is high as one example.

The output control unit 604 controls the display unit 65 to display the infection information, various messages, images, and the like. Furthermore, the output control unit 604 controls the sound output unit 66 such that message sound, an alarm, and the like are output.

By adopting the above-described configuration, the communication terminal 6 can appropriately notify the second user of a region having a high infection risk. Thus, the second user can avoid entry to and a long-term stay in a region having a high infection risk based on infection information.

The communication terminal 6 can also notify the second user of the level of the risk of the second user being infected with the virus from behaviors of the second user. The second user for which a high risk of infection risk has been calculated can know that they need to get tested for viral infection even if no symptoms have manifested yet. Thus, viral infection of the second user can be found at an early stage.

### Information Processing Device 1 and Storage Device 9

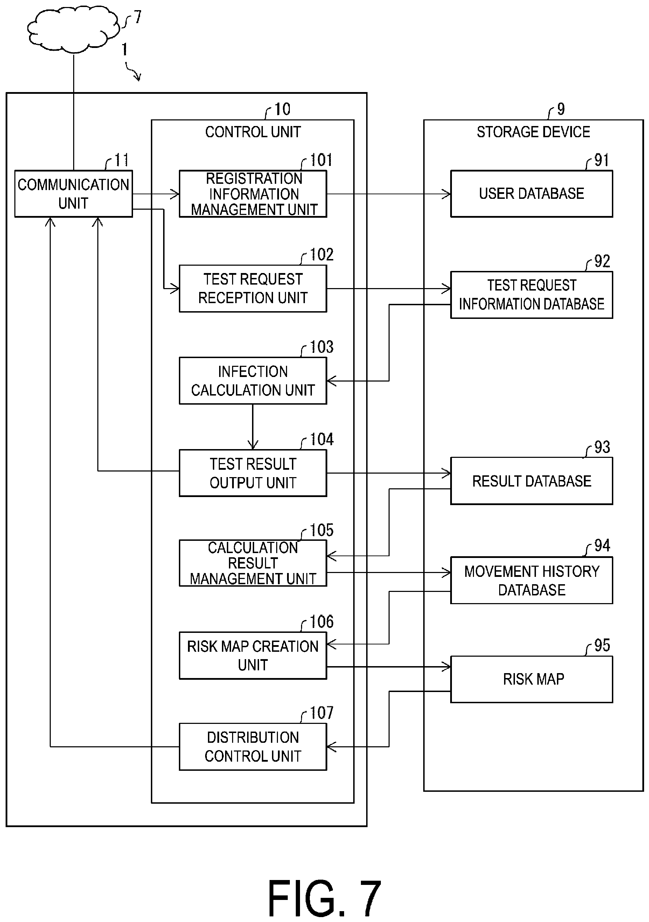

A configuration of the information processing device 1 and the storage device 9 will be described using FIG. 7. FIG. 7 is a block diagram illustrating an example of a configuration of the information processing device 1 and the storage device 9. The number of information processing devices 1 and storage devices 9 may be arbitrary, may be one as illustrated in FIG. 7, or may be plural.

### Configuration of Information Processing Device 1

The information processing device 1 generates test result information in response to reception of test request information from the communication terminal 2. The information processing device 1 transmits the generated test result information to the communication terminal 2 that is the transmission source of the test request information.

The information processing device 1 is a computer communicatively coupled to the communication terminal 2 and the storage device 9, and includes a control unit 10 that controls various processing operations executed by the information processing device 1, and a communication unit 11. The communication unit 11 performs communication with the communication terminal 2 and communication with the communication terminal 6.

The control unit 10 is a CPU, for example, and performs control to execute processing of each function of the information processing device 1. The control unit 10 includes a registration information management unit 101, a test request reception unit 102, an infection calculation unit 103, a test result output unit 104, a calculation result management unit 105, a risk map creation unit 106, and a distribution control unit 107.

The storage device 9 is a storage device in which various computer programs to be read by the control unit 10, data to be used in various processing operations executed by the control unit 10, various types of information generated by the control unit 10, and the like are stored. The storage device 9 stores a user database (user DB) 91, a test request information database (test request information DB) 92, a result database (result DB) 93, a movement history database (movement history DB) 94, a risk map 95, and the like.

The registration information management unit 101 stores registration information acquired from each user on the user DB 91 of the storage device 9 in association with personal information (including the user ID) of each user.

### User DB 91

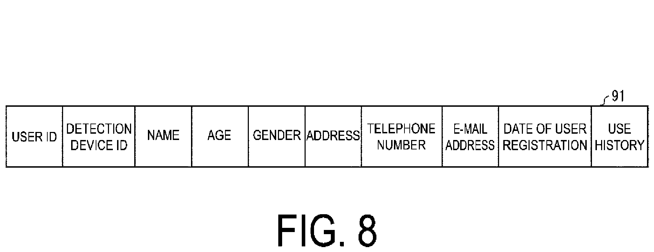

FIG. 8 is a diagram illustrating an example of a data structure of the user DB 91. In the user DB 91, personal information of each user may be associated with the user ID of the user and the detection device ID unique to the detection device 3 used by the user as illustrated in FIG. 8. Here, the personal information may be the name, age, gender, address, telephone number, e-mail address, and the like of the user.

The user DB 91 may include the user registration date on which the user submitted the registration information, and a usage history showing the usage of the test system 100 by the user. For example, the administrator of the information processing device 1 can aggregate the periods and usage frequencies for use of the test system 100 for each of users based on the user registration dates and the usage histories of the users. This allows the administrator of the information processing device 1 to prompt a user having a low usage frequency to actively use the test system 100 at an appropriate timing, for example.

Returning to FIG. 7, the test request reception unit 102 receives the test request information transmitted from the communication terminal 2. The test request reception unit 102 may give a test request information ID to each piece of received test request information, and store the ID in the test request information DB 92.

### Test Request Information DB 92

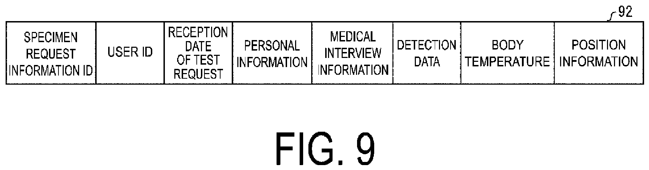

FIG. 9 is a diagram illustrating an example of a data structure of the test request information DB 92. In the test request information DB 92, the user ID, the reception date of a test request, the medical interview information, the detection data, the body temperature, and the position information may be associated with the test request information ID of each piece of the test request information as illustrated in FIG. 8.

Returning to FIG. 7, the infection calculation unit 103 analyzes the detection data included in the test request information, and calculates an infection state of the user. The infection calculation unit 103 can output the calculation result by executing an infection state calculation process. The calculation result may include "virus species", "virus count", and "determination result".

"Virus species" is information indicative of the detected major virus species. "Virus species" may be, for example, "influenza virus type *".

"Virus count" is information indicating the number of particles of the virus obtained by analyzing the detection data. As an example, "virus count" may be the number of particles of the virus detected per unit area of the specimen attachment part 321 provided with the specimen (m × 10ⁿ/cm²). Alternatively, "virus count" may be the number of particles of the virus per unit volume of the specimen (s x 10^{t}/ml).

"Determination result" is a determination result of whether the user is infected with the virus. For example, when the value indicated by "virus count" is equal to or greater than a predetermined number, the user may be determined to be "positive", and as an example, the predetermined number may be 1. Alternatively, the "determination result" may show the likelihood of the user being infected with the virus as a percentage, using a predetermined determination criterion.

The infection calculation unit 103 may generate detection information from an image taken of the specimen attachment part 321 if the test request information includes that image. The detection information may be information including the number of particles of the virus in a predetermined area on the specimen attachment part 321.

The infection calculation unit 103 may store the output calculation result, the detection information, and the like in the result DB 93.

The test result output unit 104 generates test result information including the calculation result by the infection calculation unit 103, and outputs the test result information to the communication terminal 2. The test result output unit 104 may output the detection information including the image taken of the specimen attachment part 321 or the number of particles of the virus in the predetermined region calculated from the image taken of the specimen attachment part 321 to the communication terminal.

The test result output unit 104 may generate, based on the detection information, a plot image in which the calculation result and the position of the particles of the virus detected on the specimen attachment part 321 are plotted. The test result output unit 104 may output the plot image to the communication terminal 2.

The test result output unit 104 may create infectivity information based on the detection data when the infection calculation unit 103 determines that the user is infected with the virus. Here, the infectivity information may be information indicating whether the virus carried by the user has a predetermined or higher level of infectivity with respect to a person.

The test result output unit 104 may, when the infection calculation unit 103 determines that the user is infected with the virus, estimate the infection phase for the user based on a first detection result and a second detection result and may generate infection phase information about the infection phase. Here, the infection phase information is information indicating what infection phase the user is in among a plurality of infection phases for a period from the infection state of the user being infected with the virus to a non-infection state.

The plurality of infection phases includes at least one of phases (1) to (8) below.
(1) Date of infection when the user was infected with the virus
(2) Virus incubation period
(3) Date of start of isolation when the virus carried by the user has a predetermined or higher level of infectivity enough to have a person other than the user infected
(4) Exacerbation period in which the virus count contained in the specimen collected from the user tends to increase
(5) Onset period in which symptoms developed in the user continue
(6) Recovery period in which the virus count for the specimen collected from the user tends to decrease
(7) Date of release from isolation when the infectivity of the virus carried by the user decreases to a predetermined level not sufficient to infect a person other than the user
(8) Date of complete cure when the user changes to being in a non-infection state from the infection state in which the user is infected with the virus

Furthermore, the test result output unit 104 may, when the infection calculation unit 103 determines that the user is infected with the virus, estimate the infection phase for the user based on the detection data and medical interview information and may generate infection phase information about the infection phase.

The test result output unit 104 may store the infection phase information, the plot image and the like in the result DB 93. If this configuration is adopted, the information processing device 1 can provide the user infected with the virus with information useful to the user, such as when the user was infected with the virus, when the onset of the symptoms occurred, how long the user needs to stay at home, when the infection will be completely cured, and the like.

### Test Result Information

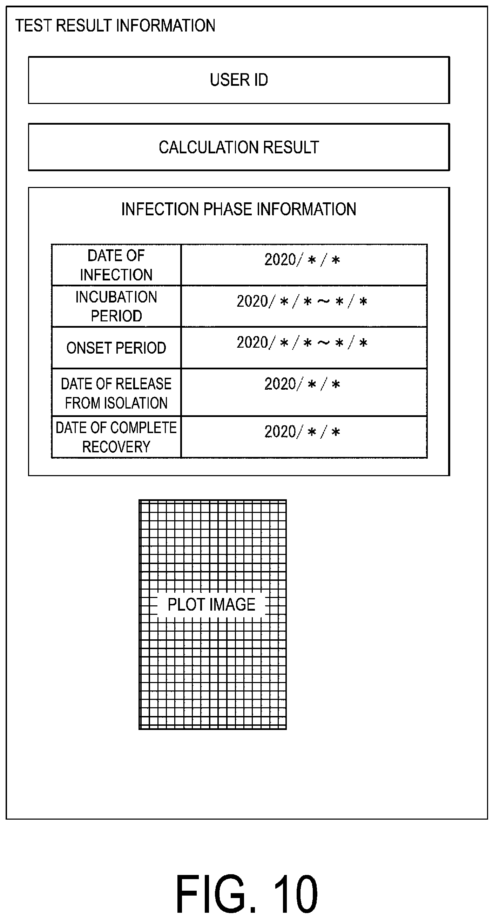

FIG. 10 is a diagram illustrating an example of the test result information. The test result information is generated when the information processing device 1 receives the test request information, and is transmitted to the communication terminal 2 that is the transmission source of the test request information. The test result information may include the calculation result, the infection phase information, and the plot image. The test result information may further include the user ID included in the test request information. Furthermore, the test result information may display a moving image or a still image associated with the calculation result or the infection phase information.

The plot image may be an image representing the position of each specimen embedding part 322 in which particles of the virus are present on the specimen attachment part 321. In the plot image shown in FIG. 10, each one of the fine squares corresponds to the position of each specimen embedding part 322. The plot image clearly shows whether the number of detected particles of virus is large or small. By providing the test result information including the plot image in this manner, the user can be effectively given a chance to start a treatment for the viral infection early. For example, even a user who has not yet developed a viral infection will be able to realize that they have been infected with the virus if they see the large number of detected particles of the virus.

### Result DB 93

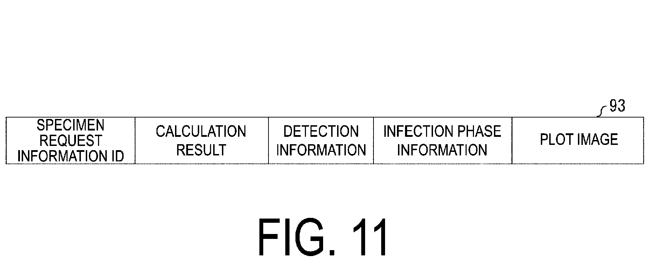

FIG. 11 is a diagram illustrating an example of a data structure of the result DB 93. In the result DB 93, the calculation result, the detection information, the infection phase information, and the plot image may be associated with each test request information ID as illustrated in FIG. 11. Alternatively, in the result DB 93, the calculation result, the detection information, the infection phase information, and the plot image may be associated with each user ID under which the test request information was transmitted.

Returning to FIG. 7, the calculation result management unit 105 stores movement history information of the user acquired from the communication terminal 2 in the movement history DB 94 in association with the calculation result and the infection phase information. The calculation result management unit 105 may store the movement history information of the user in association with the infection phase of the user estimated by the test result output unit 104 in the movement history DB 94.

### Movement History DB 94

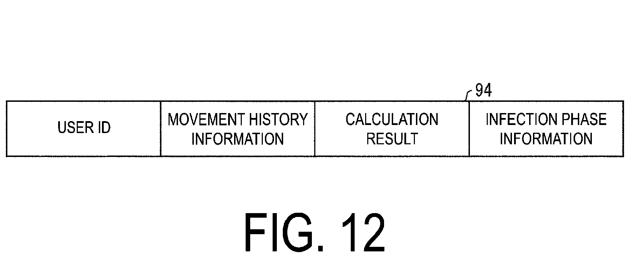

FIG. 12 is a diagram illustrating an example of a data structure of the movement history DB 94. As illustrated in FIG. 12, the movement history DB 94 may have movement history information, detection information, and infection phase information associated with the user ID of each user.

Returning to FIG. 7, the risk map creation unit 106 creates a risk map in which movement history information of each of a plurality of users determined to be infected with the virus is plotted on a map in association with the infection phase of each of the plurality of users. The risk map creation unit 106 does not utilize information with which each user can be identified, such as a user ID, among records included in the movement history DB 94, for example. In other words, the risk map creation unit 106 creates a risk map 95 by only using the calculation result, the infection phase information, and the movement history information included in the record of the user determined to be infected with the virus, for example.

Specifically, for each user with a "positive" test result, the risk map creation unit 106 plots on the map the place where the user was present at the time or time point when the user may have released the virus with infectivity, in any manner that indicates the place has a high infection risk. The risk map creation unit 106 repeats this process for a plurality of users with "positive" test results. As a result, the risk map creation unit 106 can create the risk map 95 representing places with a relatively high infection risk and places with a relatively low infection risk.

The risk map creation unit 106 may create and update the risk map 95 periodically (e.g., every one to two hours). A risk map created by the risk map creation unit 106 in the past may also be stored in the storage device 9. In this case, the risk map creation unit 106 may store the risk map 95 in the storage device 9 each time the risk map is created in association with the date and time on which the risk map 95 was created.

### Risk Map 95

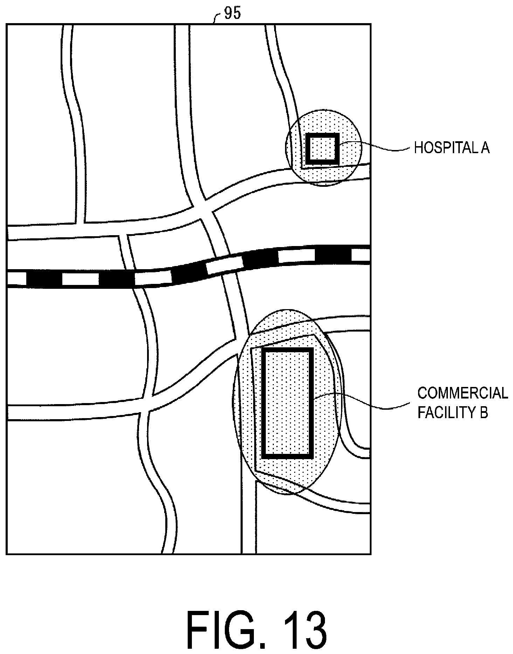

FIG. 13 is a diagram illustrating an example of the risk map 95 created by the risk map creation unit 106. In the example illustrated in FIG. 13, the area around a "hospital A" and the area around a commercial facility B are shown as places with a high infection risk. The risk map may be illustrated in the form of a "heat map" with colors representing high and low population densities, names of facilities such as "hospital A" or "commercial facility B", names of regions or street addresses indicating particular locations or areas around particular locations. The risk map may further represent a change over time. In this case, for example, a place with a relatively high infection risk may be indicated in dark red, and a place with a relatively low infection risk may be indicated in light red on the risk map 95.

Returning to FIG. 7, the distribution control unit 107 distributes infection information in response to an information distribution request from the communication terminal 6.

The information distribution request may include the user ID of the second user, and information indicating a target area for which the second user desires to acquire the infection information. In this case, the distribution control unit 107 may distribute the risk map 95 including the target area to the communication terminal 6. The information distribution request may further include information indicating the current position of the second user. In this case, the distribution control unit 107 may distribute the risk map 95 including the target area to the communication terminal 6.

The distribution control unit 107 may transmit past test result information about viral infection of the user in response to viewing request information from the communication terminal 2. The user's ID or the like may be included in the viewing request information.

### Process Flow

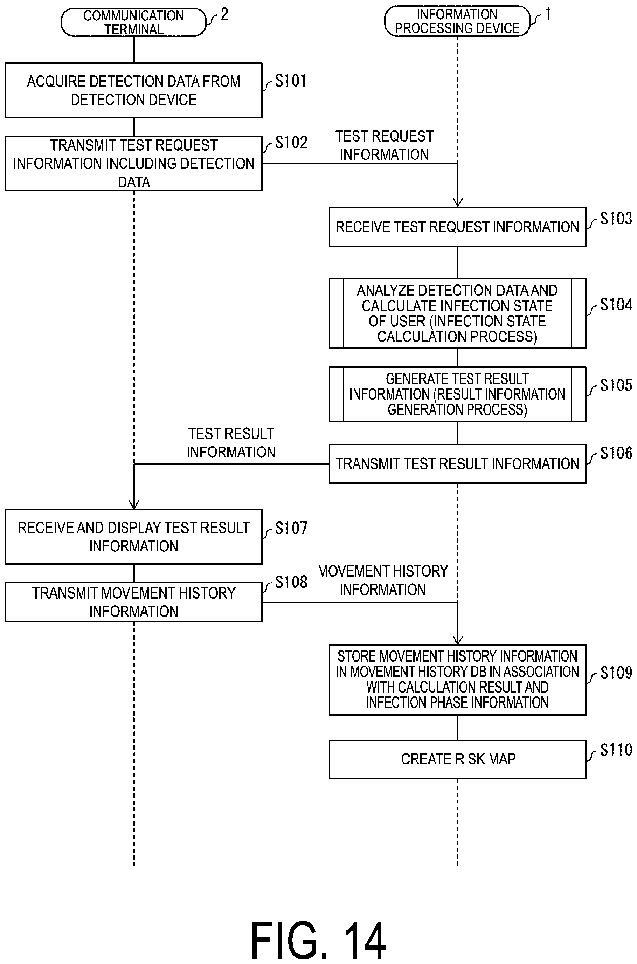

FIG. 14 is a sequence diagram showing the flow of a process of each device of the test system 100. FIG. 14 shows an example of the flow of a process in which the information processing device 1 that has received test request information from the communication terminal 2 transmits test result information and then creates the risk map 95.

In step S101, the communication terminal 2 acquires the detection data from the detection device 3. The communication terminal 2 may perform short-range wireless communication with the detection device 3 to acquire the detection data, or may acquire the detection data from the detection device 3 coupled thereto via a Universal Serial Bus (USB) terminal.

In step S102, the communication terminal 2 transmits the test request information including the detection data. In addition to the detection data, the communication terminal 2 may include, for example, the user ID, the personal information, the medical interview information, the body temperature, and the position information of the communication terminal 2 at the specimen collection date and time in the test request information. The position information may, for example, be acquired from the position information reception unit 23 of the communication terminal 2.

In step S103 (a test request reception step), the test request reception unit 102 of the information processing device 1 receives the above-described test request information received via the communication unit 11. The test request reception unit 102 may give a test request information ID to each received piece of test request information. The test request reception unit 102 stores the received test request information in the test request information DB 92 along with the reception date.

In step S104 (an infection calculation step), the infection calculation unit 103 analyzes the detection data and calculates an infection state of the user of the communication terminal 2. Hereinafter, this process will be referred to as an infection state calculation process. The infection calculation unit 103 may store the output calculation result in the result DB 93.

In step S105, the test result output unit 104 generates test result information based on various types of information included in the test request information and the output calculation result. Hereinafter, this process will be referred to as a result information generation process. The test result output unit 104 can output the test result information by executing the result information generation process. The test result output unit 104 may store the output test result information in the result DB 93. The test result information may include, for example, the calculation result output in S104, and the infection phase information output in S105.

In step S106 (a test result output step), the test result output unit 104 transmits, via the communication unit 11, the output test result information to the communication terminal 2 as the source of request.

In step S107, the communication terminal 2 receives the test result information from the information processing device 1 via the communication network 7. Then, the communication terminal 2 displays the received test result information on the display unit 25 of the communication terminal 2.

In step S108, the communication terminal 2 transmits movement history information. The movement history information is information associated with position information indicating the position where the communication terminal 2 was present, and the date and time at which the communication terminal 2 was present at that position. The position information may be, as an example, longitude/latitude information acquired from the GPS antenna of the communication terminal 2. The date and time may be the date and time at which the longitude/latitude information was acquired from the GPS antenna.

Further, the communication terminal 2 may transmit the movement history information to the information processing device 1 before transmitting the test request information. Alternatively, the communication terminal 2 may transmit the movement history information along with the test request information when the test request information is transmitted. Furthermore, the communication terminal 2 may transmit the movement history information if the test result information acquired in S 107 includes a positive determination result. In this case (i.e., if the test result information includes a positive determination result), the test result information transmitted from the information processing device 1 may include a transmission request for transmitting the movement history information, or the communication terminal 2 may transmit the movement history information in response to such a transmission request. Furthermore, the communication terminal 2 may collectively transmit the movement history information within a predetermined period from the specimen collection date (for example, a period of two weeks before the specimen collection date, etc.). Alternatively, the communication terminal 2 may periodically (e.g., every few minutes) transmit the position information indicating the current position of the communication terminal 2 as the movement history information to the information processing device 1, regardless of the timing at which the test request information is transmitted and whether the determination result is negative or positive.

In step S109 (a calculation result management step), the calculation result management unit 105 associates the user ID and the movement history information with the calculation result and the infection phase information and stores the data in the movement history DB 94.

In step S110 (a risk map creation step), the risk map creation unit 106 creates a risk map indicating the infection phase, the times, and the places that the person was in with reference to the movement history DB 94.

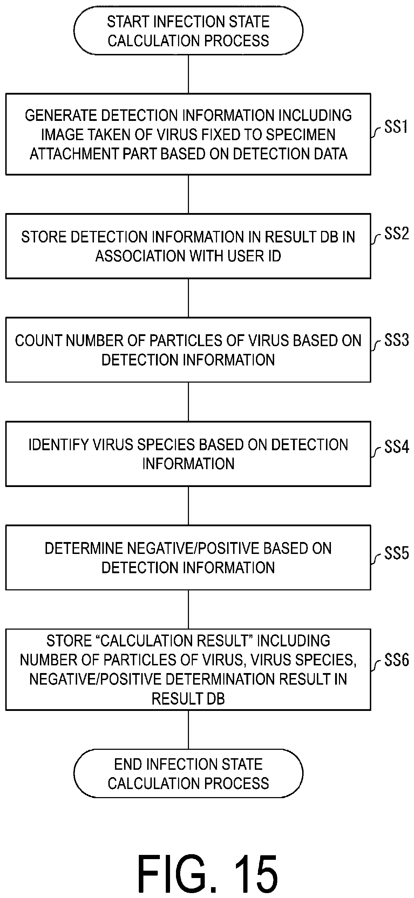

FIG. 15 is a flowchart showing an example of the flow of an infection state calculation process executed by the infection calculation unit 103.

In step SS1, the infection calculation unit 103 generates detection information including an image taken of the virus fixed to the specimen attachment part 321 based on the detection data transmitted from the communication terminal 2.

In step SS2, the infection calculation unit 103 stores the generated detection information in the result DB93 in association with the user ID.

In step SS3, the infection calculation unit 103 counts the number of particles of the virus based on the detection information.

In step SS4, the infection calculation unit 103 identifies the virus species based on the detection information.

In step SS5, the infection calculation unit 103 make a negative or positive determination based on the detection information. That is, the infection calculation unit 103 determines whether the user is infected with the virus.

In step SS6, the infection calculation unit 103 stores the calculation result including the number of particles of the virus, the virus species, and the negative or positive determination result in the result DB 93.

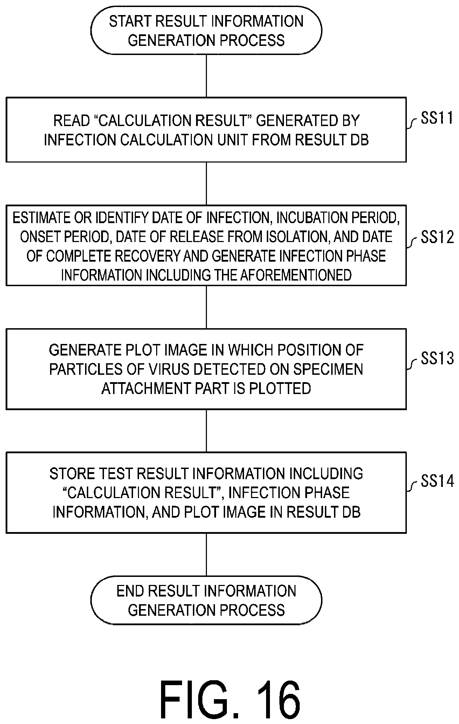

FIG. 16 is a flowchart showing an example of the flow of a result information generation process executed by the test result output unit 104.

In step SS11, the test result output unit 104 reads the above-described calculation result generated by the infection calculation unit 103 from the result DB 93.

In step SS12, the test result output unit 104 estimates or identifies the date of infection, the incubation period, the onset period, the date of isolation release, and the date of complete recovery based on the test request information, the detection information, and the calculation result. Then, the test result output unit 104 generates infection phase information including the date of infection, the incubation period, the onset period, the date of isolation release, and the date of complete recovery.

In step SS13, the test result output unit 104 generates, based on the detection information, a plot image plotting the position of the particles of the virus detected on the specimen attachment part 321.

In step SS14, the test result output unit 104 stores the test result information including the calculation result, the infection phase information, and the plot image in the result DB 93.

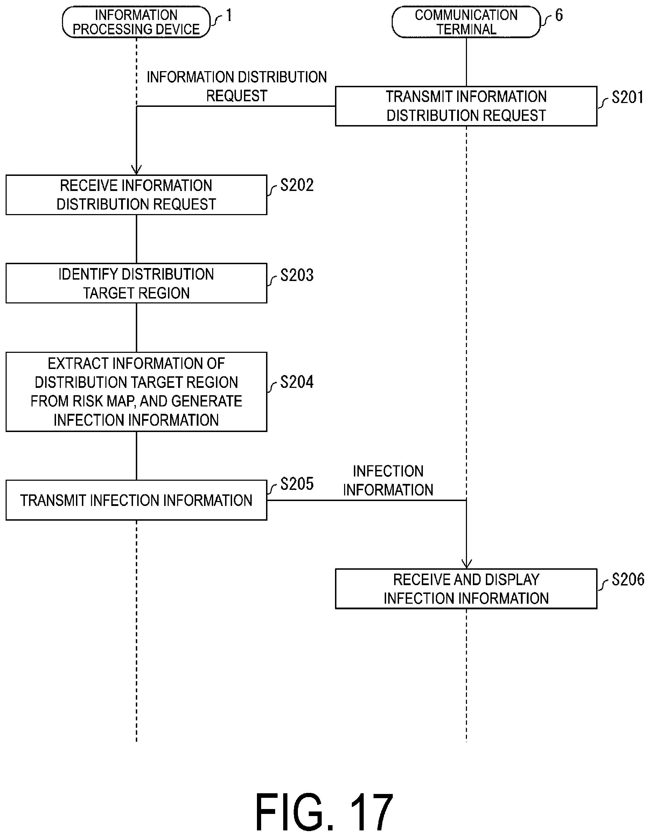

FIG. 17 is a sequence diagram showing the flow of a process of each device of the test system 100. Most of all, FIG. 17 shows an example of the flow of a process performed by the information processing device 1 to distribute the infection information to the communication terminal 6 in response to an information distribution request from the communication terminal 6.

In step S201, the communication terminal 6 transmits an information distribution request. The information distribution request is information for the communication terminal 6 to request distribution of the infection information from the information processing device 1.

In step S202, the distribution control unit 107 of the information processing device 1 receives the information distribution request from the communication terminal 6 via the communication unit 11.

In step S203, the distribution control unit 107 identifies the distribution target region based on the information distribution request. For example, the distribution control unit 107 may identify the region registered by the second user in advance (a region including the region around the address such as home or workplace) as a distribution target region based on the user ID of the second user included in the information distribution request. Alternatively, if the information distribution request includes information specifying the region desired by the second user, separately from the region registered by the user in advance, the distribution control unit 107 may identify the distribution target region based on the information. Alternatively, if the information distribution request includes the position information of the communication terminal 6 (specifically, the longitude/latitude information), the distribution control unit 107 may identify the range of the radius Nkm centered on the longitude/latitude information as a distribution target region.

In step S204, the distribution control unit 107 extracts information of the distribution target region identified in S203 from the risk map 95 stored in the storage device 9, and generates infection information.

In step S205, the distribution control unit 107 transmits the infection information generated in S204 to the communication terminal 6.

In step S206, the communication terminal 6 receives the infection information from the information processing device 1, and displays the received infection information on the display unit 65 of the communication terminal.

### Second Embodiment

Another embodiment of the present disclosure will be described below. Note that, for convenience of description, a member having the same function as that of a member described in the embodiment described above is denoted by the same reference sign, and description thereof will not be repeated.

Such test result information and the infection information generated by the information processing device 1 may be available to medical facilities, private companies, and public institutions. A test system 100a employing such a configuration will be described below.

### Configuration of Test System 100a

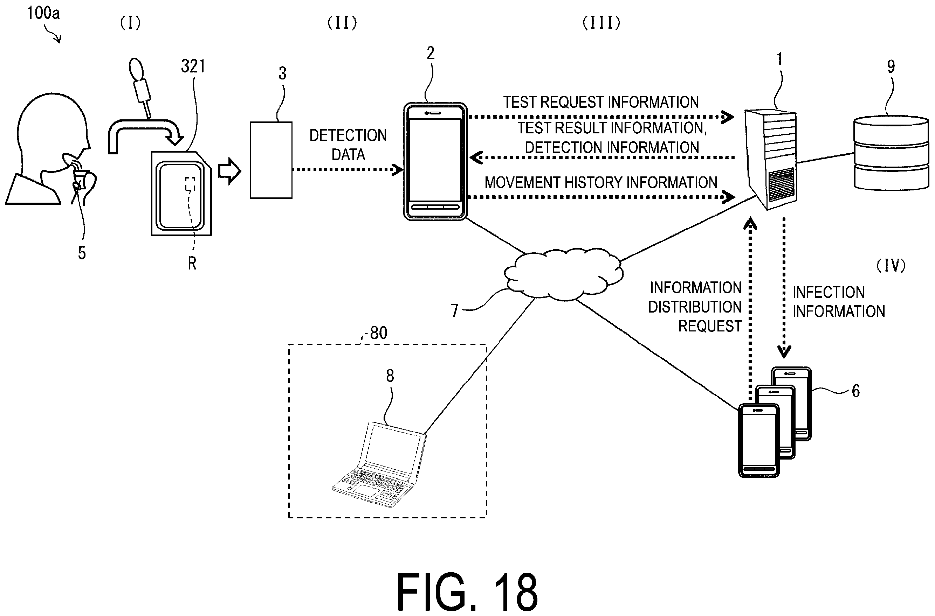

A configuration of the test system 100a according to another aspect of the present disclosure will be described using FIG. 18. FIG. 18 is a diagram illustrating a configuration example of the test system 100a according to a second embodiment of the present disclosure. Although FIG. 18 illustrates a configuration in which a medical facility terminal 8 installed in a medical facility 80 uses the test system 100a, but the disclosure is not limited thereto.

The test system 100a includes not only a detection device 3, an information processing device 1, a detection device 3, and a communication terminal 2 (and a communication terminal 6), but also the medical facility terminal 8 communicatively coupled to the information processing device 1 and the communication terminal 2. The information processing device 1, the communication terminal 2 (and the communication terminal 6), and the medical facility terminal 8 are communicatively coupled via a communication network 7.

The medical facility terminal 8 is a computer terminal used by a medical professional (e.g., a doctor) belonging to the medical facility 80 that uses the test system 100a. The medical facility terminal 8 is, for example, a personal computer, a tablet terminal, a smartphone, or the like. The medical facility terminal 8 includes a communication unit for communicating with another device, an input unit such as a keyboard or a microphone, a display unit such as a monitor, and an output unit such as a speaker.

For example, a doctor of the medical facility 80 using the test system 100a can view test request information received by the information processing device 1, test result information and infection information generated by the information processing device 1 using the medical facility terminal 8.

By using the test system 100a, the doctor of the medical facility 80, or the like can ascertain the number of virus-infected people in each region, the state of the spread of the viral infection, and the like. Each regional point may be, for example, in the vicinity of the medical facility 80. Consequently, the doctor or the like of the medical facility 80, for example, can predict in advance that there will be an increase in the number of outpatient consultations for virus-infected patients, and can alert other patients early on that the viral infection is getting prevalent.

### Example of Implementation by Software

The control blocks of the information processing device 1 (in particular, the registration information management unit 101, the test request reception unit 102, the infection calculation unit 103, the test result output unit 104, the calculation result management unit 105, and the risk map creation unit 106) may be implemented by a logic circuit (hardware) formed in an integrated circuit (an IC chip) or the like, or may be implemented by software.

In the latter case, the information processing device 1 includes a computer that executes instructions of a program that is software implementing each function. The computer includes, for example, one or more processors and a computer-readable recording medium that stores the above program. Then, in the computer, the processor reads the above program from the recording medium and executes the read program to achieve the object of the present disclosure. As the processor, a central processing unit (CPU) can be used, for example. As the recording medium, a "non-transitory tangible medium", for example, a tape, a disk, a card, a semiconductor memory, a programmable logic circuit, and the like as well as a read only memory (ROM) can be used. In addition, a random access memory (RAM) for loading the above program may be further provided. In addition, the above program may be supplied to the computer via any transmission medium (a communication network or broadcast waves) capable of transmitting the program. Further, one aspect of the present disclosure may be implemented in the form of data signals embedded in a carrier wave in which the above program is embodied by electronic transmission.

The present disclosure is not limited to each of the embodiments described above, and various modifications can be made within the scope indicated by the claims, and an embodiment obtained by appropriately combining technical means disclosed in different embodiments is also included in a technical scope of the present disclosure.

### REFERENCE SIGNS

1 Information processing device
2 Communication terminal
3 Detection device
5 Specimen
9 Storage device
100, 100a Test system
102 Test request reception unit
103 Infection calculation unit
104 Test result output unit
105 Calculation result management unit
106 Risk map creation unit
321 Specimen attachment part
S103 Test request reception step
S104 Infection calculation step
S 106 Test result output step
S 109 Calculation result management step
S 110 Risk map creation step

## Claims

1. An information processing device comprising:
a test request reception unit configured to receive, from a communication terminal, test request information including detection data obtained by a detection device configured to detect a detection target object contained in a specimen collected from a user;
an infection calculation unit configured to calculate an infection state of the user based on the detection data; and
a test result output unit configured to generate test result information including a calculation result from the infection calculation unit and output the test result information to the communication terminal.

2. The information processing device according to claim 1, wherein
the test request information includes personal information of the user.

3. The information processing device according to claim 1 or 2, wherein
the detection device includes a specimen attachment part to which the detection target object is fixed, and
the test result output unit outputs detection information including an image taken of the specimen attachment part or a number of detection target objects in a predetermined region, the number being calculated from the image, to the communication terminal.

4. The information processing device according to claim 3, wherein
the test result output unit generates, based on the detection information, a plot image in which the calculation result and a position of the detection target object detected on the specimen attachment part are plotted, and outputs the plot image to the communication terminal.

5. The information processing device according to any one of claims 1 to 4, wherein
the detection data includes a first detection result obtained by detecting the detection target object contained in a specimen collected from the user at a first time and a second detection result obtained by detecting the detection target object contained in a specimen collected from the user at a second time different from the first time.

6. The information processing device according to any one of claims 1 to 5, wherein
the test request information further includes medical interview information including information regarding a symptom developed in the user and information indicating the date of observation on which the symptom was observed.

7. The information processing device according to claim 5, wherein
when the infection calculation unit determines that the user is infected with the detection target object, the test result output unit estimates in which infection phase the user is among a plurality of infection phases set for a period from an infection state of the user being infected with the detection target object to a non-infection state based on the first detection result and the second detection result, and generates infection phase information regarding the estimated infection phase.

8. The information processing device according to claim 6, wherein
when the infection calculation unit determines that the user is infected with the detection target object, the test result output unit estimates in which infection phase the user is among a plurality of infection phases set for a period from an infection state of the user being infected with the detection target object to a non-infection state based on the detection data and the medical interview information, and generates infection phase information regarding the estimated infection phase.

9. The information processing device according to claim 7 or 8, wherein
the plurality of infection phases includes at least one of the date of infection when the user was infected with the detection target object, an incubation period of the detection target object, the date of isolation start when the detection target object carried by the user has a predetermined or higher level of infectivity enough to have a person other than the user infected, an exacerbation period in which the number of detection target objects contained in the specimen tends to increase, an onset period in which a symptom developed in the user continues, a recovery period in which the number of detection target objects contained in the specimen tends to decrease, a date of release from isolation when the detection target object carried by the user has decreasing infectivity to a predetermined level not sufficient to have a person other than the user infected, and the date of complete recovery when the user changes to being in a non-infection state from an infection state in which the user was infected with the detection target object.

10. The information processing device according to claim 8 or 9, wherein
the test request reception unit further receives movement history information indicating a movement history of the user after a predetermined period of time from a time point when the test request information was transmitted from the communication terminal,
the information processing device further comprising:
a calculation result management unit for storing the movement history information in a storage device in association with the calculation result and the infection phase information.

11. The information processing device according to claim 10, wherein
the calculation result management unit stores the movement history information of the user in the storage device in association with the infection phase of the user estimated by the test result output unit.

12. The information processing device according to claim 11, further comprising:
a risk map creation unit for creating a risk map in which movement history information of each of a plurality of users determined to be infected with the detection target object is plotted on a map in association with the infection phase of each of the plurality of users.

13. The information processing device according to any one of claims 1 to 12, wherein
the detection target object is a virus or a bacterium.

14. A test system comprising:
a detection device for detecting a detection target object contained in a specimen collected from a user;
the information processing device according to any one of claims 1 to 13; and
a communication terminal communicatively coupled to the detection device and the information processing device.

15. A control method for an information processing device, the control method comprising:
receiving, from a communication terminal, test request information including detection data obtained by a detection device configured to detect a detection target object contained in a specimen collected from a user;
calculating an infection state of the user based on the detection data; and
generating test result information including a calculation result in the infection calculation and transmitting the test result information to the communication terminal.

16. The control method according to claim 15, further comprising:
in the receiving of test request information, receiving movement history information indicating a movement history of the user after a predetermined period of time from a time point when the test request information is transmitted from the communication terminal; and
calculation result processing of storing the movement history information in a storage device in association with the calculation result.

17. The control method according to claim 16, further comprising:
risk map creation of creating a risk map in which movement history information of each of a plurality of users determined to be infected with the detection target object is plotted on a map in association with an infection phase of each of the plurality of users.

18. A control program for causing a computer to function as the information processing device according to any one of claims 1 to 13, wherein
a process executed by the information processing device includes
receiving, from a communication terminal, test request information including detection data obtained by a detection device configured to detect a detection target object contained in a specimen collected from a user;
calculating an infection state of the user based on the detection data; and
generating test result information including a calculation result by the infection calculation unit and transmitting the test result information to the communication terminal.

19. A computer readable recording medium recording the control program according to claim 18.
